# EUROPÄISCHE PATENTSCHRIFT

(11) **EP 1 263 832 B1**
(45) Veröffentlichungstag und Bekanntmachung des Hinweises auf die Patenterteilung: **28.04.2004**
(21) Anmeldenummer: 01925333.5
(22) Anmeldetag: 16.02.2001
(51) Int. Cl.: C08G 18/73, C07C 263/10, C07C 265/14, C09J 175/04, C09D 175/04

(54) **POLYISOCYANATE ALS BESCHICHTUNGSKOMPONENTEN FÜR FESTKÖRPERREICHE BESCHICHTUNGSMITTEL**
POLYISOCYANATES AS COATING COMPONENTS FOR COATING MEANS RICH IN SOLIDS
POLYISOCYANATES UTILISES COMME COMPOSANTS DE REVETEMENT POUR PRODUITS DE REVETEMENT RICHES EN SOLIDES

(30) Priorität: 16.02.2000 DE 10006902; 16.02.2000 DE 10006901
(43) Veröffentlichungstag der Anmeldung: 11.12.2002
(73) Patentinhaber: BASF AKTIENGESELLSCHAFT, 67056 Ludwigshafen (DE)
(72) Erfinder: HENKELMANN, Jochem, 68165 Mannheim (DE); MAAS, Heiko, 68165 Mannheim (DE); SCHULZ, Gerhard, 67098 Bad Dürkheim (DE); STAMM, Armin, 55128 Mainz (DE); RINK, Heinz-Peter, 48153 Münster (DE); JUNG, Werner-Alfons, 59387 Ascheberg (DE); SCHWAB, Peter, 67098 Bad Dürkheim (DE)
(74) Vertreter: Isenbruck, Günter, Dr.
(86) Internationale Anmeldenummer: PCT/EP2001/001784
(87) Internationale Veröffentlichungsnummer: WO 2001/060885

(56) Entgegenhaltungen:
- EP-A- 0 013 493
- EP-A- 0 749 958
- EP-A- 0 928 799
- DATABASE WPI Section Ch, Week 198304 Derwent Publications Ltd., London, GB; Class A25, AN 1983-08094K XP002172241 & JP 57 200419 A (ASAHI CHEM IND CO LTD), 8. Dezember 1982 (1982-12-08)

## Beschreibung

Die Erfindung betrifft aliphatische Polyisocyanate, ein Verfahren zu ihrer Herstellung, die aliphatischen Polyisocyanate enthaltende Beschichtungsmittel und Klebstoffe sowie die Verwendung der Polyisocyanate, Beschichtungsmittel und Klebstoffe.

Aliphatische Polyisocyanate, das heißt Isocyanate mit mehr als einer, bevorzugt mehr als zwei Isocyanatgruppen im Molekül, sind bekannt und werden unter anderem als Vernetzer zur Herstellung von Polyurethanen eingesetzt, die als Bindemittel für Lacke geeignet sind. Da die Herabsetzung des Lösemittelgehalts von Lacken zur Verminderung von Emissionen angestrebt wird, ist es zur Einhaltung einer für das Aufbringen des Lacks ausreichend niedrigen Viskosität notwendig, Lackbestandteile mit niedriger Viskosität zur Verfügung zu haben. Handelsübliche Polyisocyanate, die diese Bedingung erfüllen, werden in der Regel durch Oligomerisieren von zweiwertigen Isocyanaten, zum Beispiel von Hexamethylendiisocyanat, hergestellt. Bei dieser Herstellung wird jedoch ein Teil der ursprünglich vorhandenen Isocyanatgruppen umgesetzt und geht für die spätere Vernetzungsreaktion verloren. Außerdem erfordert dieses Herstellungsverfahren zwei Stufen bis zum mehrwertigen Isocyanat mit drei oder mehr NCO-Gruppen.

Andere mehrwertige Isocyanate, zum Beispiel das Nonantriisocyanat, werden direkt aus den entsprechenden mehrwertigen Aminen hergestellt. Von solchen mehrwertigen Aminen sind aber nur wenige Vertreter im technischen Maßstab verfügbar.

Heute bekannte Lacksysteme, wie Klar- oder Decklacke, Füller und Grundierungen oder Mittel für den Unterbodenschutz von Kraftfahrzeugen, weisen den Nachteil auf, daß ihre Festkörpergehalte nicht unbegrenzt erhöht werden können. Solche Lacksysteme sind aus den DE 44 07 415, DE 44 07 409 oder DE 43 10 414 bekannt. Die Anhebung des Festkörpergehaltes von Lacken zur Reduktion der Lösungsmittelemission ist jedoch ein erklärtes Ziel der Lackindustrie. Dies gilt sinngemäß auch für Klebstoffe und Dichtmassen.

Aufgabe der Erfindung ist es, neue aliphatische Polyisocyanate, die bei niedriger Viskosität mehrere NCO-Gruppen im Molekül enthalten und deren Molekülgröße und Anzahl an NCO-Gruppen sich nach Wunsch variieren läßt, sowie ein Verfahren zu ihrer Herstellung zur Verfügung zu stellen, das nach Einführung der NCO-Gruppen keine weitere Umsetzung erfordert.

Aufgabe der vorliegenden Erfindung ist ferner, Beschichtungsmittel oder Klebstoffe bereitzustellen, die gegenüber den bisher bekannten Beschichtungsmitteln einen erhöhten Festkörpergehalt aufweisen und emissionsarme Formulierungen ermöglichen.

Gelöst wird die Aufgabe durch Polyisocyanate der allgemeinen Formel I 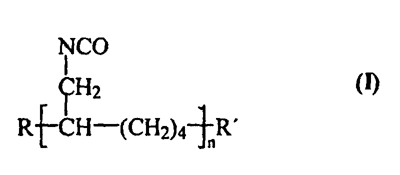 worin
- R und R': gleiche oder verschiedene Alkylgruppen mit 1 - 4 C-Atomen bedeuten und
- n: eine Zahl von im Mittel 1,5 bis 5 ist.

Die Aufgabe wird ferner gelöst durch ein Beschichtungsmittel oder einen Klebstoff, enthaltend
a) Polyisocyanate der allgemeinen Formel I, wobei die Polyisocyanate in blockierter Form vorliegen können, als Vernetzer
b) gegebenenfalls weitere Vernetzer und
c) isocyanatreaktive Polymere, Oligomere und/oder niedermolekulare Verbindungen als Bindemittel.

Polyisocyanate der allgemeinen Formel I sind somit solche Verbindungen, die im Mittel 1,5 bis 5 NCO-Gruppen enthalten, d.h. im wesentlichen solche Vertreter, die als Oligomere bezeichnet werden können. Die Polyisocyanate der allgemeinen Formel I liegen häufig als Gemische von Homologen vor, die Vertreter mit n = 1 bis etwa n = 10 enthalten und vorzugsweise im wesentlichen aus diesen Vertretern bestehen.

Erfindungsgemäß wird ferner ein Verfahren zur Herstellung von Polyisocyanaten der allgemeinen Formel 1 vorgeschlagen, das darin besteht, daß man ein Polyamin der Formel II 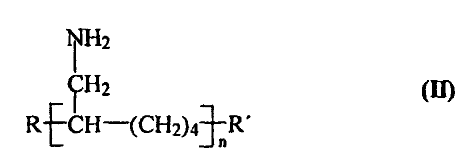 worin R, R' und n die oben angegebene Bedeutung haben, mit Phosgen, vorzugsweise im Überschuß, umsetzt.

Die Umsetzung der Polyamine kann einstufig bei erhöhter Temperatur (im allgemeinen von 120 bis 170°C) oder besonders vorteilhaft zweistufig, zunächst bei niedrigeren Temperaturen im Bereich von - 15 bis + 10°C (Kaltphosgenierung) und anschließender Erhöhung auf 130 bis 165°C (Heißphosgenierung), durchgeführt werden. Besonders gute Ausbeuten werden erzielt, wenn das Amin zunächst in das Hydrochlorid überführt wird und anschließend einer Kaltphosgenierung bei 50 bis 70°C und einer Heißphosgenierung bei 120 bis 170°C unterworfen wird. Phosgen wird im allgemeinen in einem Überschuß von 10 bis 30 Mol-% eingesetzt, so daß üblicherweise im wesentlichen alle primären Aminogruppen zu Isocyanatgruppen umgesetzt werden. Man kann aber auch größere Phosgenüberschüsse einsetzen. Das überschüssige Phosgen kann auf einfache Weise entfernt und in die Synthese zurückgeführt werden.

Die Umsetzung kann ohne Zusatz von inerten Lösungsmitteln durchgeführt werden. Im allgemeinen ist es jedoch von Vorteil, in einem aprotischen Lösungsmittel wie einem halogenierten, insbesondere chlorierten aromatischen, vorzugsweise einkernigen Kohlenwasserstoff wie Dichlorbenzol, zu arbeiten.

Die Verbindungen der Formel II sind bekannt und beispielsweise in der DE-A 196 54 167 beschrieben. Sie werden bevorzugt durch katalysierte Metathesereaktion von Cyclopenten enthaltenden Kohlenwasserstoffgemischen, z.B. Erdölfraktionen, anschließender Hydroformylierung der Reaktionsprodukte und nachfolgende Umsetzung mit Ammoniak in Gegenwart von Wasserstoff und einem Hydrierungskatalysator erhalten.

Die erhaltenen Polyisocyanate zeichnen sich durch besonders niedrige Viskositäten aus, die im allgemeinen unterhalb von 400 mPas liegen. Sie sind damit hervorragend zur Herstellung von lösungsmittel armen Lackrohstoffgemischen geeignet.

Gegenstand der vorliegenden Erfindung ist auch die Verwendung der Polyisocyanate der allgemeinen Formel 1 zur Herstellung von Beschichtungs-mitteln und Klebstoffen.

Die Durchschnittsfunktionalität der erfindungsgemäß verwendeten Isocyanate beträgt vorzugsweise von 1,5 bis 5,0 und besonders bevorzugt von 1,5 bis 3,5.

Die Polyisocyanate der allgemeinen Formel I können als alleinige Vernetzer oder in Abmischungen mit weiteren Vernetzern eingesetzt werden. Beispiele geeigneter weiterer Vernetzer sind Aminoplastharze, Siloxangruppen enthaltende Verbindungen oder Harze, Anhydridgruppen enthaltende Verbindungen oder Harze, Polyisocyanate und/oder Alkoxycarbonylaminotriazine.

Vorzugsweise enthalten die weiteren Vernetzer weitere, von den Polyisocyanaten der allgemeinen Formel 1 verschiedene Polyisocyanate oder bestehen aus diesen. Prinzipiell können alle auf dem Lackgebiet üblichen Polyisocyanate eingesetzt werden. Bevorzugt sind Polyisocyanate, deren Isocyanatgruppen an aliphatische oder cycloaliphatische Reste gebunden sind. Beispiele sind Tetramethylendiisocyanat, Hexamethylendiisocyanat, Isophorondiisocyanat, Trimethylhexamethylendiisocyanat, Dicyclohexylmethandiisocyanat, 1,3-Bis(2-isocyanatopropyl-2)-benzol (TMXDI), 1,4- und 1,3-Bis(isocyanatomethyl)-cyclohexan und andere sowie Addukte dieser Polyisocyanate an Polyole und von diesen Polyisocyanaten abgeleitete isocyanuaratgruppen- und/oder biurethgruppenhaltige Polyisocyanate.

Die Polyisocyanate der allgemeinen Formel I und die als weitere Vernetzer eingesetzten Polyisocyanate können in blockierter oder unblockierter Form vorliegen.

Durch Wahl der weiteren Vernetzer sowie durch Einsatz der Polyisocyanate in blockierter oder unblockierter Form können die erfindungsgemäßen Beschichtungsmittel und Klebstoffe als Ein- oder Zweikomponenten-Systeme ausgelegt werden.

Sowohl in Ein- als auch in Zweikomponenten-Systemen können blockierte Isocyanate zum Einsatz kommen, vorzugsweise kommen sie in Einkomponenten-Systemen zum Einsatz. Die Herstellung geeigneter blockierter Isocyanate ist beispielsweise in der DE-A 198 09 643 allgemein beschrieben. Vorzugsweise enthalten blockierte Isocyanate sowohl mit einem ersten Blockierungsmittel als auch mit einem zweiten Blockierungsmittel blockierte Isocyanatgruppen, wobei das erste Blockierungsmittel ein Dialkylmalonat oder eine Mischung aus Dialkylmalonaten, vorzugsweise Dialkylmalonate mit 1 - 6 C-Atomen und das zweite Blockierungsmittel vorzugsweise ein Acetessigsäurealkylester mit 1 - 6 C-Atomen im Alkylrest oder ein Ketoxim, wie Acetessigsäureethylester oder Methylethylketoxim ist. Vorzugsweise liegt das Verhältnis zwischen den mit dem ersten Blockierungsmittel und den mit dem zweiten Blockierungsmittel blockierten Isocyanatgruppen zwischen 8,0 : 2,0 und 6,0 : 4,0, wobei geeignete blockierte Polyisocyanate erhältlich sind durch Umsetzung der Polyisocyanate mit einer Mischung aus dem ersten und dem zweiten Blockierungsmittel oder durch Mischen von mit dem ersten Blockierungsmittel blockierten Isocyanaten und mit dem zweiten Blockierungsmittel blockierten Polyisocyanaten in den entsprechenden Verhältnissen.

Geeignete blockierte Polyisocyanate werden auch erhalten, indem ein Teil, vorzugsweise 70 % der Isocyanatgruppen des Polyisocyanats mit einer Mischung aus dem ersten und dem zweiten Blockierungsmittel umgesetzt wird und die verbleibenden Isocyanatgruppen mit einer hydroxylgruppenhaltigen Verbindung umgesetzt werden.

Bei den Einkomponentensystemen können die Polyisocyanate in blockierter Form zusätzlich in Abmischung mit Aminoplastharzen eingesetzt werden.

Die erfindungsgemäßen Beschichtungsmittel und Klebstoffe können Zweikomponenten-Systeme sein. In diesem Fall weist das Beschichtungsmittel oder der Klebstoff eine zweite Komponente auf, die als Vernetzer ein gegebenenfalls in einem organischen Lösungsmittel gelöstes nicht blockiertes Polyisocyanat enthält. Zusätzlich können blockierte Polyisocyanate in der zweiten Komponente vorhanden sein. Bei den nicht blockierten Polyisocyanaten handelt es sich um nicht blockierte, erfindungsgemäß verwendete Isocyanate der allgemeinen Formel I und gegebenenfalls - bei Vorliegen weiterer Vernetzer - um beliebige weitere nicht blockierte organische Polyisocyanate mit aliphatisch, cycloaliphatisch, araliphatisch und /oder aromatisch gebundenen, freien Isocyanatgruppen. Beispiele für weitere Polyisocyanate sind isocyanatgruppenhaltige Polyurethanprepolymere, die durch Reaktion von Polyolen mit einem Überschuß an Polyisocyanaten hergestellt werden können und die bevorzugt niedrigviskos sind. Es können auch Isocyanuratgruppen, Biurethgruppen, Allophanatgruppen, Urethangruppen, Harnstoffgruppen, Urethdiongruppen und/oder Iminooxadiazindiongruppen aufweisende Polyisocyanate als weitere Polyisocyanate eingesetzt werden. Derartige Polyisocyanate sind ebenfalls in der DE-A 198 09 643 beschrieben.

Außerdem können aliphatische. Triisocyanate wie 1,8-Diisocyanato-4-isocyanatomethyloctan, 1,7-Diisocyanato-4-isocyanatomethylheptan und/oder Tris(alkoxycarbonylamino)triazin als weitere Vernetzer eingesetzt werden.

Im allgemeinen liegen die Polyisocyanate der allgemeinen Formel I in dem Gesamtvemetzer in Anteilen von 5 bis 100 Gew.-%, vorzugsweise von 10 bis 80 Gew.-%, besonders bevorzugt von 20 bis 70 Gew.-%, d. h. bezogen auf die Summe der Polyisocyanate der allgemeinen Formel I und der weiteren Vernetzer, vor. Ebenso können die Polyisocyanate der Formel I in isocyanatfunktionelle Derivate Urethane, Allophanate, Isocyanurate usw. oder Mischungen davon umgewandelt und eingesetzt werden.

Die erfindungsgemäßen Beschichtungsmittel und Klebstoffe enthalten isocyanatreaktive Polymere, Oligomere und/oder niedermolekulare Verbindungen als Bindemittelkomponente. Es kommen alle hydroxy- und aminofunktionellen sowie sonstige isocyanatreaktive Polymere, Oligomere und niedermolekulare Verbindungen in Betracht. Vorzugsweise enthält die Bindemittelkomponente hydroxyfunktionelle Polymere, Oligomere und/oder niedermolekulare Verbindungen, besonders bevorzugt besteht sie aus diesen. Hydroxyfunktionelle Polymere und/oder Oligomere sind vorzugsweise ausgewählt aus der Gruppe bestehend aus hydroxyfunktionellen Polyacrylaten, Polyestern, Polyurethanen, acrylierten Polyurethanen, acrylierten Polyestern, Polylactonen, Polycarbonaten, Polyethern und (Meth)acrylatdiolen. Vorzugsweise werden Polyacrylate, Polyester und/oder Polyurethane, insbesondere Polyacrylate und/oder Polyester eingesetzt. Geeignete Polyacrylate, Polyester und Polyurethane sind in der DE-A 198 09 643 beschrieben.

Geeignete isocyanatreaktive niedermolekulare Verbindungen sind beispielsweise die in der DE-A 198 09 643 beschriebenen verzweigten, cyclischen und/oder acyclischen C₉ - C₁₆-Alkane, die mit mindestens 2 Hydroxyl- oder Thiolgruppen oder mindestens einer Hydroxyl- und mindestens einer Thiolgruppe funktionalisiert sind. Von diesen sind die C₉ - C₁₆ - Alkanpolyole bevorzugt. Besonders bevorzugt sind die stellungsisomeren Dialkyloctandiole, insbesondere Diethyloctandiol, speziell 2,4-Diethyloctandiol-1,5. Die genannten isocyanatreaktiven niedermolekularen Verbindungen können in der Bindemittelkomponente beispielsweise in Anteilen von 0,5 bis 25 Gew.-%, bezogen auf die Gesamtmenge des Bindemittels, enthalten sein.

Ferner kann das erfindungsgemäße Beschichtungsmittel UV-Absorber, Radikalfänger, Katalysatoren für die Vernetzung, Rheologiemittel, Verarbeitungszeitverlängerer sowie weitere übliche Additive wie Slipadditive, Polymerisationsinhibitoren, Mattierungsmittel, Entschäumer, Verlaufsmittel und filmbildende Hilfsmittel, z.B. Cellulose-Derivate, oder andere, in Basislacken üblicherweise eingesetzte Additive enthalten. Als Vemetzungskatalysatoren kommen insbesondere metallorganische Verbindungen, vorzugsweise Zinn und/oder wismutorganische Verbindungen, in Betracht. In Frage kommen ferner tertiäre Amine. Verarbeitungszeitverlängerer (pot life additives) sind beispielsweise Acetylaceton und tertiäre Alkohole wie tert.-Butanol. Es können Pigmente jeglicher Art, beispielsweise Farbpigmente wie Azopigmente, Phthalocyaninpigmente, Carbonylpigmente, Dioxazinpigmente, Titandioxid, Farbruß, Eisen-, Chrom- und Kobaltoxide, oder Effektpigmente wie Metallplättchenpigmente, insbesondere Aluminiumplättchenpigmente und Perlglanzpigmente, Verwendung finden.

Die Herstellung der erfindungsgemäßen Beschichtungsmittel und Klebstoffe erfolgt nach üblichen Methoden durch Zusammengeben der einzelnen Bestandteile und ihrem Vermischen unter Rühren.

Das erfindungsgemäße Beschichtungsmittel oder der erfindungsgemäße Klebstoff sind vorzugsweise als nicht wässrige Lösung oder Dispersion formuliert. Hierfür können die in der Lack- oder Klebstoffherstellung üblichen organischen Lösungsmittel Verwendung finden.

Das erfindungsgemäße Beschichtungsmittel wird vor allem zur Herstellung von beschichteten Formteilen oder Verbundteilen, welche Folien, Glas, Holz, Papier und/oder Metall enthalten oder hieraus bestehen. durch Auftragen des Beschichtungsmittels auf den entsprechenden Formteilen und Aushärten der resultierenden Beschichtung verwendet.

Das erfindungsgemäße Beschichtungsmittel wird vorzugsweise zur Herstellung von Ein- oder Mehrschichtlackierungen und besonders bevorzugt zur Herstellung von Decklacken eingesetzt. Es kann aber auch zur Herstellung eines über einer Basislackschicht zu applizierenden Klarlacks, beispielsweise eines Klarlacks einer nach dem Naß-in-Naß-Verfahren hergestellten Mehrschichtlackierung bestimmt sein. Darüber hinaus kann es auch als Grundierung, Füller oder Unterbodenschutz verwendet werden. Die Kunststoffe oder anderen Substrate können auch direkt mit dem Klarlack oder dem Decklack beschichtet werden.

Die Beschichtungsmittel können sowohl bei der Serien- als auch bei der Reparaturlackierung von Automobilkarosserien eingesetzt werden. Sie werden aber bevorzugt im Bereich der Serienlackierung eingesetzt.

Gegenstand der vorliegenden Erfindung ist auch die Verwendung der erfindungsgemäßen Beschichtungsmittel in der Autoserien- und Autoreparaturlackierung als Klarlack, Decklack, Füller, Grundierung und/oder Unterbodenschutz.

Der erfindungsgemäße Klebstoff dient zur Herstellung von verklebten Verbundteilen, welche Folien, Kunststoffe, Glas, Holz, Papier und/oder Metall enthalten oder hieraus bestehen, indem der Klebstoff auf der oder den zu verklebenden Oberflächen einer Folie oder eines Kunststoff, Glas, Holz, Papier und/oder Metall enthaltenden oder daraus bestehenden Formteils und/oder der Oberfläche des Teils, das hiermit verklebt werden soll, aufgetragen und gegebenenfalls vorgehärtet wird, wonach die betreffenden zu verklebenden Oberflächen in Kontakt gebracht werden und anschließend der Klebstoff ausgehärtet wird.

Die Applikation erfolgt im allgemeinen mit Hilfe üblicher Methoden, beispielsweise durch Spritzen, Rakeln, Tauchen oder Streichen.

Gegenstand der vorliegenden Erfindung ist auch die Verwendung der erfindungsgemäßen Beschichtungsmittel oder Klebstoffe zum Bestreichen und Verkleben von Kunststoff-, Holz-, Glas-, Papier- oder Metalloberflächen.

Die erfindungsgemäßen Beschichtungsmittel oder Klebstoffe werden vorzugsweise bei Temperaturen von Raumtemperatur bis zu 180°C gehärtet. Besonders bevorzugt sind Temperaturen von 60°C bis 180°C. In speziellen Anwendungsformen der erfindungsgemäßen Beschichtungsmittel können auch tiefere Härtungstemperaturen von 60°C bis 160°C angewendet werden.

Die folgenden Beispiele beschreiben bevorzugte Ausführungsformen der Erfindung. Die darin angegebenen Isocyanatzahlen werden nach DIN 16945 und 53185 durch Umsetzung des Isocyanats mit überschüssigem Dibutylamin und Rücktitration des Überschusses mit Salzsäure gegen Bromphenolblau bestimmt. Die Aminzahl ist definiert als mg KOH/g Produkt. Zur Bestimmung werden ca. 1,0 g Prüfsubstanz exakt eingewogen, in 50 ml Essigsäure gelöst und mit 0,1 molarer Triflourmethansulfonsäurmaßlösung in Essigsäure potentiographisch titriert. Eine Beschreibung der Methode findet sich in Huber, Titrationen in nichtwäßrigen Lösungsmitteln, AkademischeVerlagsgesellschaft, Frankfurt a. M., S. 130 ff. (1964) und in Gyenes, Titrationen in nichtwäßrigen Medien, Ferdinand Enke Verlag, Stuttgart, S. 488 ff. (1970).

### Beispiel 1

In einem 11 fassenden Glasrührkolben mit Gaseinleitungsrohr, der in einem Solekühler von -10°C und einem Trockeneiskühler gekühlt wurde, wurden bei -10 bis 0°C 130 g Phosgen vorgelegt. Dazu wurden innerhalb von 5 Stunden 400 g mehrwertiges Amin entsprechend der Formel II mit der Aminzahl 235,3 und weitere 470 g Phosgen gegeben. Danach wurde 1 Stunde bei -10 bis 0°C nachgerührt. Die Reaktionsmischung wurde auf 150 - 160°C erwärmt, und innerhalb von 7 Stunden wurden weitere 136 g Phosgen eingeleitet. Danach wurde bei 150°C so lange Stickstoff durch die Reaktionsmischung geleitet, bis alles Phosgen entfernt war. Es wurden 437 g eines mehrwertigen Isocyanats mit der Isocyanatzahl 12,7 g je 100 g erhalten.

### Beispiel 2

In der in Beispiel 1 angegebenen Apparatur wurden 270 g Phosgen, gelöst in 600 g o-Dichlorbenzol, bei -10 - 0°C vorgelegt und 150 g eines Polyamins mit der Aminzahl 235,3 innerhalb von 3 Stunden bei -10°C bis 0°C zugetropft. Danach wurde das Gemisch auf 140 - 145°C erwärmt, und es wurden weitere 32 g Phosgen innerhalb von 2 Stunden eingeleitet. Nach 3 Stunden Nachreaktion wurde das restliche Phosgen mit Stickstoff entfernt. Das Lösemittel wurde bei 0,5 mbar und 100°C Badtemperatur über eine Brücke abdestilliert. Es wurden 131 g Polyisocyanat mit der Isocyanatzahl 13,8 erhalten.

### Beispiel 3

Wie in Beispiel 2 beschrieben, wurden in einer Glasrührapparatur 100 g Polyamin mit der Aminzahl 306 innerhalb von 2 Stunden bei -10 bis 0°C zu einer Lösung von 170 g Phosgen in 200 ml o-Dichlorbenzol getropft. Dann wurde 4 Stunden bei 0°C nachgerührt und anschließend auf 135 - 140°C erwärmt. Innerhalb von 2 Stunden wurden weitere 30 g Phosgen eingeleitet. Nach 5 Stunden Nachreaktion bei 135 - 140°C wurde mit Stickstoff phosgenfrei gestrippt und das Lösemittel wie in Beispiel 2 beschrieben abdestilliert. Es wurden 110 g eines Polyisocyanats mit einer Isocyanatzahl von 21,9 erhalten.

### Beispiel 4

In der in Beispiel 1 beschriebenen Apparatur wurden bei -10 bis 0°C 340 g Phosgen, gelöst in 400 ml o-Dichlorbenzol, vorgelegt und 200 g Polyamin mit der Aminzahl 306 wurden innerhalb von 4 Stunden bei der gleichen Temperatur zugetropft. Nach 4 Stunden Nachreaktion bei 0 - 5°C wurde das Gemisch auf 140°C erwärmt, und innerhalb von 2 Stunden wurden weitere 60 g Phosgen eingeleitet. Nach 3 Stunden Nachreaktion bei 140°C wurde mit Stickstoff phosgenfrei gestrippt und das Lösemittel wie in Beispiel 2 beschrieben abdestilliert. Es wurden 220 g eines Polyisocyanats mit einer Isocyanatzahl von 21,3 erhalten.

### Beispiel 5

In einer wie in Beispiel 1 beschriebenen Apparatur wurden 100 g Polyamin (Aminzahl 306) vorgelegt (in 200 ml o-Dichlorbenzol) und bei Raumtemperatur bis zur Sättigung Chlorwasserstoff eingeleitet. Dabei fiel ein weißer, kristalliner Niederschlag an. Danach wurde auf 60°C aufgeheitzt und innerhalb von 6 Stunden 170g Phosgen eingeleitet. Danach wurde das Gemisch auf 145°C aufgeheitzt und weitere 60 g Phosgen eingeleitet (Dauer 2 Stunden). Danach wurde wie in Beispiel 2 beschrieben aufgearbeitet. Es wurden 110 g Polyisocyanat mit einer Isocyanatzahl von 24,7 erhalten.

### Beispiel 6

Die nachstehenden Komponenten wurden in den angegebenen Gewichtsverhältnissen homogen vermischt:
62,1 Gew.-% einer 57,4 gew.-%igen Lösung eines hydroxyfunktionellen Styrol-Polymerisats mit einer OH-Zahl von 155 und einer Glasübergangstemperatur nach Fox von 66 °C in Solventnaphtha® (einem handelsüblichen Gemisch aromatischer Kohlenwasserstoffe mit einem Siedepunktsbereich von 160 bis 185 °C) /Methoxypropylacetat / Butylacetat / Butylglykolacetat im Gewichtsverhältnis 33 : 5 : 6 : 3, als Komponente A;
0,6 Gew.-% UV-Absorber auf Basis von Triazin (Cyagard® 1164 L der Fa. Cytek) als Komponente B;
0,9 Gew.-% HALS-Stabilisatoren (Tinuvin® 292 der Fa. Ciba Geigy) als Komponente C;
0,2 Gew.-% Polysilanmodifiziertes Silikonadditiv (Byk® 310 der Fa. Byk) als Komponente D;
6,6 Gew.-% Solventnaphtha als Komponente E;
4,4 Gw.-% Butylacetat als Komponente F;
3,4 Gew.-% Butyldiglykolacetat als Komponente G;
14,3 Gew.-% eines Hexamethylendiisocynat-Trimerisats (Desmodur® N3390 der Fa. Bayer) als Komponente H;
19,9 Gew.-% eines Polyisocyanats der allgemeinen Formel I mit einer Viskosität von 364 mPas und einem NCO-Gehalt von 9 Gew.-%, einem Massenmittel Mₙ = 879 und einem Gewichtsmittel M_{w} = 2630, hergestellt aus Cyclopenten-Oligomeren, als Komponente I.

Das so erhaltene Beschichtungsmittel lieferte nach Applikation und Trocknen des Lackfilms bei 130 °C über einen Zeitraum von 30 min Beschichtungen mit guten Oberflächeneigenschaften.

## Patentansprüche

1. Polyisocyanate der allgemeinen Formel I 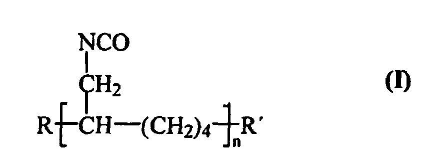 worin
R und R' gleich oder verschieden sind und Alkylgruppen mit 1 - 4 C-Atomen bedeuten und
n eine Zahl von im Mittel 1,5 bis 5 ist.

2. Polyisocyanate nach Anspruch 1, **dadurch gekennzeichnet, daß** sie aus einem Gemisch von Homologen mit n = 1 bis n = 10 bestehen.

3. Polyisocyanate nach Anspruch 1 oder 2, **dadurch gekennzeichnet, daß** sie eine Viskosität unterhalb von 400 mPas haben.

4. Verfahren zum Herstellen von Polyisocyanaten der allgemeinen Formel 1 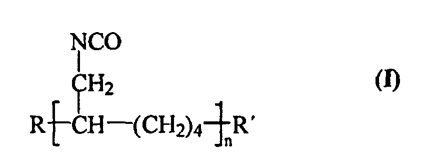 worin
R und R' gleiche oder verschiedene Alkylgruppen mit 1 - 4 C-Atomen sind und
n eine Zahl von im Mittel 1,5 bis 5 ist,
**dadurch gekennzeichnet, daß** man ein Polyamin der Formel II 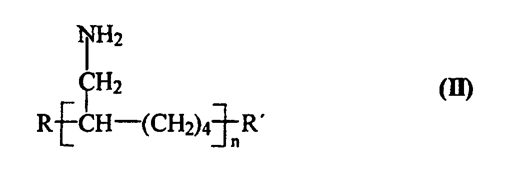 worin R, R' und n die vorstehend angegebene Bedeutung haben, mit Phosgen umsetzt.

5. Beschichtungsmittel oder Klebstoff, enthaltend
a) Polyisocyanate der allgemeinen Formel I, wie sie in der einem der Ansprüche 1 bis 3 definiert sind, wobei die Polyisocyanate in blockierter Form vorliegen können, als Vernetzer,
b) gegebenenfalls weitere Vernetzer und
c) isocyanatreaktive Polymere, Oligomere und/oder niedermolekulare Verbindungen als Bindemittel.

6. Beschichtungsmittel oder Klebstoff nach Anspruch 5, **dadurch gekennzeichnet, daß** der Anteil der Polyisocynate der allgemeinen Formel I an der Gesamtvernetzermenge von 5 Gew.-% bis 100 Gew.-% beträgt.

7. Beschichtungsmittel oder Klebstoff nach Anspruch 5 oder 6, **dadurch gekennzeichnet, daß** die weiteren Vernetzer blockierte oder nicht blockierte weitere Polyisocyanate enthalten.

8. Beschichtungsmittel oder Klebstoff nach einem der Ansprüche 5 bis 7, **dadurch gekennzeichnet, daß** das Bindemittel hydroxyfunktionelle Polymere, Oligomere und/oder niedermolekulare Verbindungen enthält.

9. Beschichtungsmittel oder Klebstoff nach Anspruch 8, **dadurch gekennzeichnet, daß** die Bindemittel ausgewählt sind aus der Gruppe bestehend aus hydroxyfunktionellen Polyacrylaten, Polyestern, Polyurethanen, acrylierten Polyurethanen, acrylierten Polyestern, Polylactonen, Polycarbonaten, Polyethern, (Meth)acrylatdiolen, und C₉-C₁₆-Alkanpolyolen.

10. Verwendung von Polyisocyanaten der allgemeinen Formel 1, wie sie in einem der Ansprüche 1 bis 3 definiert sind, zur Herstellung von Beschichtungsmitteln oder Klebstoffen.

11. Verwendung von Beschichtungsmitteln oder Klebstoffen, wie sie in einem der Ansprüche 6 bis 9 definiert sind, zum Beschichten und Verkleben von Kunststoff-, Holz-, Glas-, Papier- oder Metalloberflächen.

12. Verwendung von Beschichtungsmitteln, wie sie in einem der Ansprüche 6 bis 9 definiert sind, in der Autoserien- und Autoreparaturlackierung als Klarlack, Decklack, Füller, Grundierung und/oder Unterbodenschutz.

## Claims

1. A polyisocyanate of the formula I 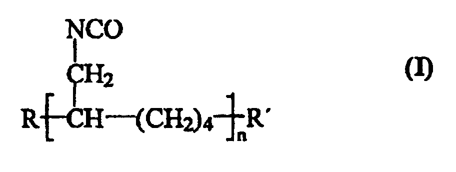 in which
R and R' are identical or different and are alkyl groups having 1-4 carbon atoms and
n is on average from 1.5 to 5.

2. A polyisocyanate as claimed in claim 1, **characterized in that** it consists of a mixture of homologs with n = 1 to n = 10.

3. A polyisocyanate as claimed in claim 1 or 2, **characterized in that** it has a viscosity of below 400 mPas.

4. A process for preparing polyisocyanates of the formula I 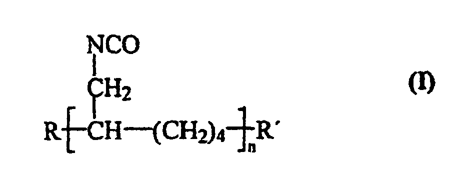 in which
R and R' are identical or different alkyl groups having 1-4 carbon atoms and
n is on average from 1.5 to 5,
**characterized in that** a polyamine of the formula II 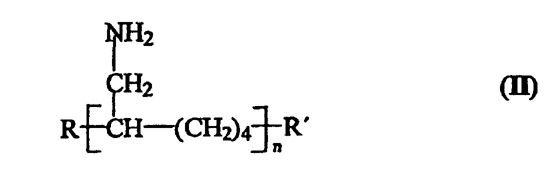 in which R, R', and n are as defined above is reacted with phosgene.

5. A coating composition or adhesive comprising
a) polyisocyanates of the formula I as defined in one of claims 1 to 3, it being possible for the polyisocyanates to be in blocked form, as crosslinkers,
b) if desired, further crosslinkers, and
c) isocyanate-reactive polymers, oligomers and/or low molecular mass compounds, as binders.

6. A coating composition or adhesive as claimed in claim 5, **characterized in that** the fraction of the polyisocyanates of the formula I in the overall crosslinker amount is from 5% by weight to 100% by weight.

7. A coating composition or adhesive as claimed in claim 5 or 6, **characterized in that** the further crosslinkers comprise blocked or nonblocked further polyisocyanates.

8. A coating composition or adhesive as claimed in one of claims 5 to 7, **characterized in that** the binder comprises hydroxy-functional polymers, oligomers and/or low molecular mass compounds.

9. A coating composition or adhesive as claimed in claim 8, **characterized in that** the binders are selected from the group consisting of hydroxy-functional polyacrylates, polyesters, polyurethanes, acrylated polyurethanes, acrylated polyesters, polylactones, polycarbonates, polyethers, (meth)acrylatediols, and C₉-C₁₆ alkanepolyols.

10. The use of polyisocyanates of the formula I as defined in one of claims 1 to 3 to prepare coating compositions or adhesives.

11. The use of coating compositions or adhesives as defined in one of claims 6 to 9 to coat and bond surfaces of plastic, wood, glass, paper or metal.

12. The use of coating compositions as defined in any of claims 6 to 9 in automotive OEM finishing and automotive refinish as a clearcoat, topcoat, surfacer, primer and/or underbody protectant.

## Revendications

1. Polyisocyanates de la formule générale I : 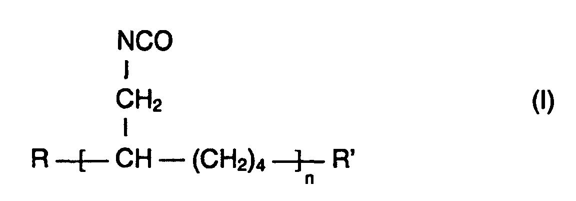 dans laquelle
R et R' sont identiques ou différents et représentent des groupes alkyle comportant 1 à 4 atomes de C, et
n est un nombre de 1,5 à 5 en moyenne.

2. Polyisocyanates suivant la revendication 1, **caractérisés en ce qu'**il sont constitués d'un mélange d'homologues où n = 1 à n = 10.

3. Polyisocyanates suivant l'une des revendications 1 et 2, **caractérisés en ce qu'**ils ont une viscosité inférieure à 400 mPas.

4. Procédé de préparation de polyisocyanates de la formule générale I : 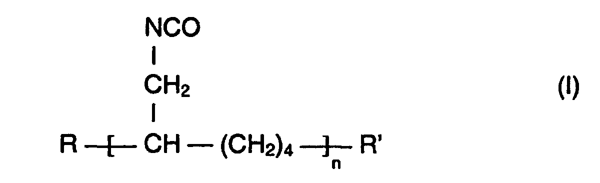 dans laquelle
R et R' sont des groupes alkyle comportant 1 à 4 atomes de C identiques ou différents, et
n est un nombre de 1,5 à 5 en moyenne,
**caractérisé en ce qu'**on fait réagir avec du phosgène une polyamine de la formule II : 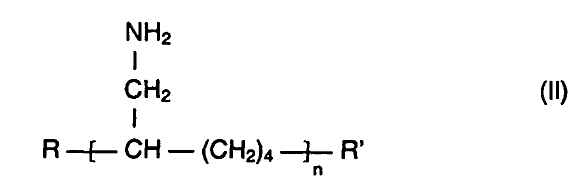 dans laquelle R, R' et n ont la signification indiquée précédemment.

5. Produit d'enduction ou substance adhésive, contenant
a) des polyisocyanates de la formule générale I, tels que ceux définis dans l'une des revendications 1 à 3, les polyisocyanates pouvant se présenter sous une forme bloquée, à titre d'agent de réticulation,
b) éventuellement d'autres agents de réticulation, et
c) des polymères, oligomères et/ou composés de faible poids moléculaire réactifs avec des isocyanates, comme liants.

6. Produit d'enduction ou substance adhésive suivant la revendication 5, **caractérisé en ce que** la fraction des polyisocyanates de la formule générale I est de 5% en poids à 100% en poids par rapport à la quantité totale d'agent de réticulation.

7. Produit d'enduction ou substance adhésive suivant l'une des revendications 5 et 6, **caractérisé en ce que** les autres agents de réticulation contiennent d'autres polyisocyanates bloqués ou non bloqués.

8. Produit d'enduction ou substance adhésive suivant l'une des revendications 5 à 7, **caractérisé en ce que** le liant contient des polymères, oligomères et/ou composés de faible poids moléculaire hydroxyfonctionnels.

9. Produit d'enduction ou substance adhésive suivant la revendication 8, **caractérisé en ce que** les liants sont choisis parmi le groupe constitué des polyacrylates, polyesters, polyuréthannes, polyuréthannes acrylés, polyesters acrylés, polylactones, polycarbonates, polyéthers, diols (méth)acryliques et alcanepolyols en C₉-C₁₆ hydroxyfonctionnels.

10. Utilisation de polyisocyanates de la formule générale I, tels que ceux définis dans l'une des revendications 1 à 3, pour la fabrication de produits d'enduction ou de substances adhésives.

11. Utilisation de produits d'enduction ou de substances adhésives, tels que ceux définis dans l'une des revendications 6 à 9, pour l'enduction et le collage de surfaces en matière synthétique, en bois, en verre, en papier ou en métal.

12. Utilisation de produits d'enduction, tels que ceux définis dans l'une des revendications 6 à 9, dans le vernissage de séries d'autos et dans la réparation d'autos, en tant que vernis clair, vernis final, charge, couche de fond et/ou protection du bas de caisse.
